(19) **Europäisches Patentamt**

**European Patent Office**

**Office européen des brevets**

(11) **EP 1 048 324 A2**

(12) **EUROPEAN PATENT APPLICATION**

(43) Date of publication:
02.11.2000 Bulletin 2000/44

(51) Int. Cl.[7]: **A61N 1/378**

(21) Application number: 00107926.8

(22) Date of filing: 14.04.2000

(84) Designated Contracting States:
**AT BE CH CY DE DK ES FI FR GB GR IE IT LI LU MC NL PT SE**
Designated Extension States:
**AL LT LV MK RO SI**

(30) Priority: 30.04.1999 US 302895

(71) Applicant: **MEDTRONIC, INC.**
**Minneapolis, Minnesota 55432-3576 (US)**

(72) Inventors:
- **Schallhorn, Richard S.**
  **Lake Elmo, MN 55042 (US)**
- **Skime, Robert M.**
  **Coon Rapids, MN (US)**

(74) Representative:
**Hughes, Andrea Michelle**
**Frank B. Dehn & Co.,**
**European Patent Attorneys,**
**179 Queen Victoria Street**
**London EC4V 4EL (GB)**

(54) **Medical Li+ rechargeable powered implantable stimulator**

(57) The implantable stimulator 1 of the present invention, having a rechargeable lithium ion power source 2, delivers electrical stimulation pulses, in a controlled manner, to a targeted site 4, 5, 6, 7 within a patient. This lithium ion power source, contained within the implantable stimulator, can supply sufficient power to the implantable stimulator on an exclusive basis over at least about 4 days, the power source including a high value, small size lithium ion storage unit having a power rating of at least 50 milliamp hours. The implantable stimulator of the present invention also has an inductor 11 adapted to gather EMF power transmissions. The implantable stimulator can be replenished with electrical power by an electrical power replenisher, external to the implantable stimulator, to replenish the lithium ion power source up to its maximum rated voltage by generating the EMF power transmissions near the inductor.

FIG. 1

EP 1 048 324 A2

**Description**

This invention relates to a medical implantable generator for electrical stimulation (such as neurostimulation) that is powered by a Lithium Ion (Li+) based rechargeable power source.

Medical implantable stimulators are used to treat patients suffering from various ailments. For example, implanted neurostimulators are used to treat patients suffering from such ailments as chronic pain, movement disorders, and incontinence. Implantable stimulators with replenishable, high capacitive power sources are known, such as U.S. Patent No. 5,807,397, assigned to Plexus, Inc. Capacitive power sources in these prior implantable stimulators must be replenished frequently on a daily or other periodic basis via an external, RF coupled device. This required frequent replenishment of capacitive power sources gives rise to a host of disadvantages, such as the frequent and continuous need for an available external, RF coupled device to replenish the capacitive power source, as well as the inconvenience of frequent replenishment of the capacitive power source. Thus, a rechargeable power source for implantable stimulators that requires less frequent replenishment than prior capacitive power sources would be desirable.

In addition, conventional implantable stimulators have a certain size and weight to account for their capacitive power sources. Thus, an implantable stimulator having a smaller size and weight, yet still providing operating times equivalent to or better than conventional capacitive powered stimulators would also be desirable.

Thus, it is an object of the present invention to provide an implantable stimulator having a rechargeable power source that requires less frequent replenishment than conventional implantable stimulators. It is a further object of the present invention to provide an implantable stimulator having a rechargeable power source that can deliver more frequent, greater, and more prolonged stimulation than conventional implantable stimulators, and thereby improve the quality of patient lives. Another object of the present invention is that the power source of the implantable stimulator can power an implantable 12 cubic centimeter neurostimulator for up to about 30 days without recharging.

It is also an object of the present invention to provide an implantable stimulator having a smaller size and lower weight than conventional capacitive powered stimulators, yet still providing equivalent or better operating times.

In addition, it is an object of the present invention to provide an implantable stimulator that can be as small as three (3) to (6) cubic centimeters, and thus provide for anatomical placement of implantable stimulators not available in conventional capacitive powered stimulators.

It is a further object of the present invention to provide an implantable stimulator that can be readily replenished via an external, RF coupled device during a shorter replenishing cycle than conventional implantable stimulators.

The invention provides a medical system for delivering electrical stimulation pulses, in a controlled manner, to a targeted site within a patient comprising:

an implantable stimulator capable of delivering, in a controlled manner, one or more electrical stimulation pulses to a targeted site within a patient;
the implantable stimulator having a rechargeable lithium ion power source sufficient to supply power to the implantable stimulator on an exclusive basis.

A new medical implantable stimulator has thus now been discovered that has a power source that requires less frequent replenishment than the capacitive power sources in conventional implantable stimulators. This new implantable stimulator has a Lithium ion (Li+) rechargeable power source, as opposed to the capacitive power sources in conventional implantable stimulators. The medical implantable stimulator of the present invention delivers more frequent, greater, and more prolonged stimulation than conventional implantable stimulators, thereby greatly improving patient lives. More specifically, the implantable stimulator of the present invention can provide up to 10 to 100 times more stimulation time per given volume than conventional implantable stimulators.

The medical implantable stimulator of the present invention can be used as a neurostimulator or any other implantable system requiring self-contained power, e.g. pacemakers, defibrillators, and cochlear implants. For example, the implantable stimulator of the present invention can provide up to at least 300 milliamp hours of stimulation. However, a range of about 10 milliamp hour to 2 amp hour Li+ rechargeable power source sizes are feasible if desired. For smaller implantable stimulators, such as cochlear implants, the implantable stimulator of the present invention can provide up to at least 50 milliamp hours of stimulation.

In a preferred embodiment of the present invention, the implantable stimulator having a Li+ rechargeable power source can power a 12 cubic centimeter neurostimulator for up to about 30 days without recharging.

Another embodiment of the present invention provides an implantable stimulator having equivalent or better operating times than conventional capacitive powered stimulators while being smaller and lighter than such conventional capacitive powered stimulators.

Another preferred embodiment of the present invention provides an implantable stimulator that can be as small as three (3) to (6) cubic centimeters, and thus provide for anatomical placement of implantable stimulators not available in conventional capacitive powered stimulators.

The implantable stimulator of the present invention can be a neurostimulator such as Itrel® II, Itrel® 3, Synergy® and Kinetra® (all from Medtronic, Inc. of Minneapolis, Minnesota) that is modified so as to have a Li+ rechargeable power source, as opposed to a capacitive power source.

Preferred embodiments of the invention will now be described, by way of example only, with reference to the drawings.

Figure 1 illustrates an implantable stimulator according to the present invention, as implanted in a preferred location of the human body.

Figure 2 illustrates locations where the implantable stimulator of the present invention can be implanted in the human body other than the location shown in Figure 1.

Figure 3 illustrates the relationship between voltage and electrical power capacity for a Li+ rechargeable power source of the present invention.

Li+ power sources, such as used in non-medical devices, e.g. laptop computers, were evaluated for a number of parameters to determine whether such power sources could be modified for use as a power source in medical implantable stimulators. The parameters reviewed were:

(1) total energy stored by each Li+ power source from full charge to full discharge;
(2) capacity fade rate as a function of cycle;
(3) capacity fade rate as a function of time;
(4) capacity fade rate as a function of storage temperature;
(5) charge rate capability; and
(6) discharge current capability (impedance current).

The evaluation of the above parameters revealed that Li+ power sources, not previously used in medical implantable stimulators, could be suitable for a power source in medical implantable stimulators upon appropriate modification. More specifically, it has been discovered that such Li+ power sources, such as commercially available Sony US2430 Cell (from Sony Corporation), could be modified by hermetic sealing the Li+ ion power source to eliminate problems arising from out-gassing and to shape the Li+ ion power source so that it can be placed within a medical implantable stimulator.

A comparison was made between the time between replenishing a Li+ power source (i.e., Sony US2430 Cell) and a capacitive power source as taught in U.S. Patent No. 5,807,397. This comparison is set forth below:

TABLE I

| Implantable Stimulator Device Size (cubic centimeters; "cc") | Weight of Implantable Stimulator Device (grams; "gm") | Approximate Time between Required Replenishing for Capacitive Power Source* | Approximate Time between Required Replenishing for Li+ Power Source* | Li+ Power Source Milliamp Hours |
|---|---|---|---|---|
| 3 to 6 cc | # 12 gm | 6 hours | 4 days | 50 milliamp hours |
| 6 to 12c c | # 25 gm | 12 hours | 30 days | 150 milliamp hours |
| 12 to 26 cc | # 40 gm | 24 hours | 60 days | 300 milliamp hours |

\* Where typical depletion current is between 100 and 150 microamps.

The above table was prepared using actual test data of a 26 cc device having a capacitive power source as taught in U.S. Patent No. 5,807,397. The remaining information in the above table is based an extrapolation of the actual performance of this 26 cc device and from evaluating a Li+ power source (i.e., Sony US2430 Cell). The relationship between the time required for replenishing a power source for a given depletion current of 150 microamps for a given stimulator device size is as follows:

1. Power Source Density ("$PS_{DEN}$")

$PS_{DEN}$ (Li+) - 50 Milliamp hours/cubic centimeters

for Li+ power source

$$PS_{DEN} (CAP) - 0.5 \text{ Milliamp hours/cubic centimeters}$$

for Capacitive power source

2. Power Source Capacity (in milliamp hours) is based on average device current and approximate time between required replenishing.

$$PS_{cap} (\text{Milliamp hours}) = T_{Repl.} * I_{Device}$$

$PS_{cap}$ = Power Source Capacity (milliamp hours)
$T_{Repl.}$ = Time between replenishing (hours)
$I_{Device}$ = Device current (milliamps)

3. Power Source Size

$$PS_{size(cc)} = PS_{CAP}/PS_{DEN}$$

$PS_{size}$ = Size of the power source (cubic centimeters)

4. Device Size

$$\text{Device Size (cubic centimeters)} = PS_{size} * S_{scale}$$

$S_{scale}$ = The proportion of the remaining device components with respect to the Power Source. Typically, the remaining device components are 3-6 times the size of the power source.

It was further determined a hermetic power cell could be constructed to eliminate the concern of out gassing, which is a reliability concern, to the surrounding electronics. For example, a suitable metal alloy can be used as an outer housing for the power cell to hermetically seal the power cell. More specifically, the outer housing can comprise two metal alloy parts that are welded together at their edges to encase the power cell. A hermetic feed through wire can be used to account for polarity.

It was further determined that in the present invention, the inductor needed to be different from that taught in U.S. Patent No. 5,807,397 to account for the greater energy stored in the Li+ cell over that stored in a conventional capacitative power sources used in the implantable stimulator of U.S. Patent No. 5,807,397. More specifically, the present invention requires an inductor that will increase the coefficient of coupling to increase the energy transfer rate due to the greater energy stored in the Li+ cell. In a preferred embodiment of the present invention, this is accomplished by a high permeable material, coincident shapes to facilitate coupling, and a coefficient coupling feedback indicator.

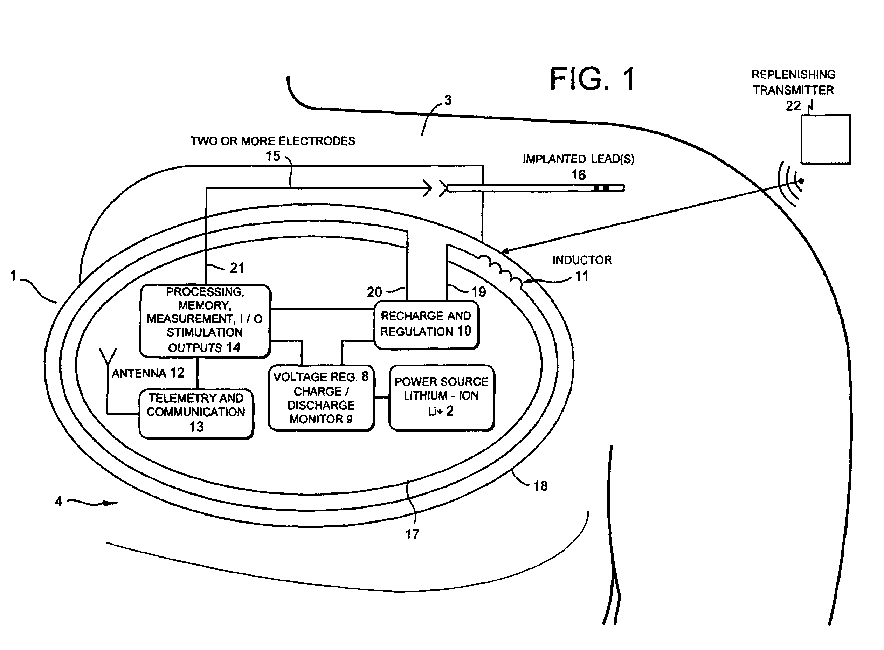

As shown in Figure 1, a new medical implantable stimulator 1 has now been discovered that has a rechargeable lithium ion power source 2, which requires less frequent replenishment than the capacitive power sources in conventional implantable stimulators. More specifically, this new stimulator has a rechargeable Lithium ion (Li+) power source 2, as opposed to the capacitive power sources in the implantable stimulator as taught in U.S. Patent No. 5,807,397. The medical implantable stimulator 1 of the present invention delivers more frequent, greater, and more prolonged stimulation than conventional implantable stimulators, thereby greatly improving patient lives. More specifically, the implantable stimulator of the present invention can provide up to 10 to 100 times more stimulation time per given volume than conventional implantable stimulators.

Figure 1 illustrates implantable stimulator 1 implanted subcutaneously with respect to a patient's skin 3. More specifically, Figure 1 shows that in one preferred embodiment, stimulator 1 is implanted in upper left side chest region 4 of the patient in a pocket just below the collar bone, and more specifically, the pectoral region.

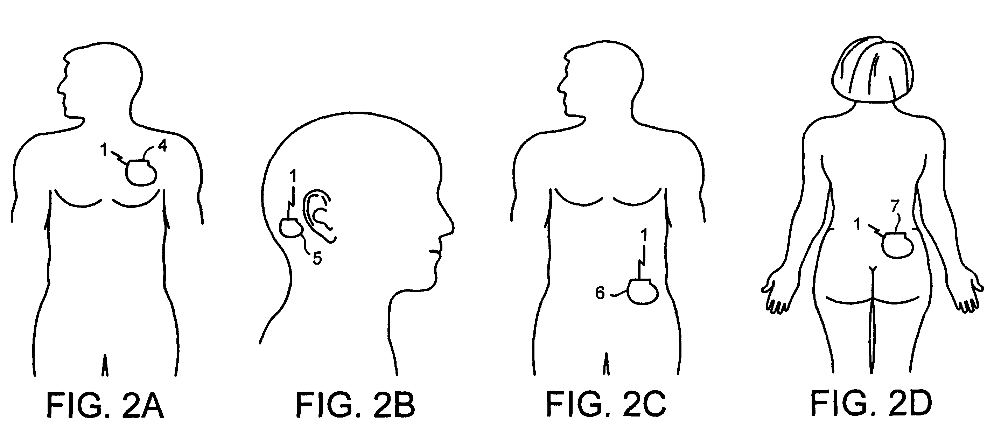

The above preferred embodiment for the placement of stimulator 1 within a human patient in pectoral region 4 is further shown in Figure 2A. Other preferred embodiments for the placement of stimulator 1 within a human patient is further shown in Figures 2B, 2C, and 2D. As shown in Figure 2B, stimulator 1 can be implanted in a region 5 behind the ear of a patient, and more specifically in the mastoid region. As shown in Figure 2C, stimulator 1 can be implanted in the lower left abominable region 6 of a patient. As shown in Figure 2D, stimulator 1 can be placed in the lower back or upper buttock region 7 of a patient. Because implantable stimulator 1 of the present invention having a Li+ power source can be smaller than an implantable stimulator having a capacitive power source, the implantable stimulator 1 of the present invention can be implanted in a patient in more locations and/or used in more applications than prior art stimulators.

Referring again to Figure 1, stimulator 1 comprises Li+ power source 2, voltage regulator 8 having charge/discharge monitor 9, recharge and regulation circuitry 10, inductor 11, antenna 12, telemetry and communication circuitry 13, and processor 14 comprising processing, memory, measurement, input/output, and stimulation output circuitry, and two (2) or more electrodes 15 having at least one implanted lead 16.

The stimulator 1 can be coupled with a programming unit (not shown) and/or a replenish transmitter 22 via radio frequency waves when they are placed in close proximity to stimulator 1. More specifically, stimulator 1 can receive command signals from such programming unit or replenish transmitter 22 via antenna 12. Programming units and replenish transmitters are known in the art. Preferably, the programming unit and the replenish transmitter 22 are not used concurrently.

Antenna 12 is coupled to telemetry and communication circuitry 13, which in turn is coupled to processor 14. Processor 14 is coupled to two (2) or more electrodes 15. Processor 14 is also coupled to voltage regulator 8 and recharge and regulation circuitry 10. Voltage regulator 8 is coupled to Li+ power source 2. Voltage regulator 8 is also coupled to recharge and regulation circuitry 10. Recharge and regulation circuitry 10 is further coupled to inductor 11. As shown in the preferred embodiment of Figure 1, stimulator 1 has an inner housing 17. Preferably, inner housing 17 is hermetically sealed and made from appropriate material, such as titanium and titanium alloys. Stimulator 1 also has an outer housing 18 containing the inner housing 17.

As shown in Figure 1, preferably inductor 11 can be placed within the outer housing 18 and outside inner housing 17. As further shown in Figure 1, inductor 11 is preferably coupled to recharge and regulation circuitry via at least two (2) separate hermetic feed through wires 19 and 20. This construction provides a larger inductor aperture than if the inductor was placed inside inner housing 17. The larger the inductor aperture, the greater the magnetic field that can be coupled into the inductor 11 and the greater the current to replenish the Li+ power source 2 of stimulator 1. Thus, this construction having the inductor 11 outside the inner housing 17 increases the coupling coefficient of implantable stimulator 1 of the present invention. Further, by placing inductor 11 outside inner housing 17, energy losses attributed to inner housing 17 are reduced. Those skilled in the art will recognize that inductor 11 can be a coil that extends around the outside of inner housing 17. If desirable, antenna 12 can also be placed within the outer housing 18 and outside inner housing 17.

Li+ power source 2 supplies voltage regulator 8 with electrical power. The electrical power is then supplied from the voltage regulator 8 to processor 14. Processor 14 then supplies electrical stimulation pulses to two (2) or more electrodes 15 via a lead connector 21. Electrodes 15 then supply the electrical stimulation pulses to implanted lead 16. Implanted lead 16 can be placed adjacent or near a targeted site within the human patient. For example, implanted lead 16 can be placed near targeted tissue, such as nerves in the spinal cord, heart tissue, or any muscle(s) where electrical stimulation is desirable.

In an alternative, Li+ power source 2 can be used to power an implantable diagnostic device to detect bodily conditions of certain organs, like the brain or heart. In another alternative, Li+ power source 2 can be used to power a drug delivery system.

In operation, the replenish transmitter 22 is placed in close proximity to skin 3 and implantable stimulator 1. An inductor (not shown) of the replenish transmitter 22 emits RF waves establishing EMF wave fronts which are received by inductor 11.

Further, replenish transmitter 22 emits command signals to antenna 12 of implantable stimulator 1. These received command signals are demodulated by telemetry and communication circuitry 13 and then communicated to processor 14. Processor 14 activates the proper control and the inductor 11 receives the RF coupled power from the inductor of the replenish transmitter 22.

The RF coupled power, which is alternating current ("AC"), is converted by the recharge and regulation circuitry 10 into a high direct current ("DC") voltage. Voltage regulator 8 converts the high DC voltage to a lower precise DC voltage while Li+ rechargeable power source 2 refreshes and replenishes. In a preferred embodiment of an implantable stimulator 1 being about 6 to 12 cc in size, approximately 150 milliamp hours are stored in Li+ rechargeable power source 2. This is sufficient to power implantable stimulator 1 (about 6 to 12 cc in size) for at least 30 days during the delivery of substantially continual electric stimulation pulses to targeted tissues via implanted lead 16. Longer energy storage times, exceeding 30 days, are possible dependent upon the value of the capacity of Li+ rechargeable power source 2 and the power drain due to the continual delivery of stimulation pulses. Because of the greater energy storage times, the present invention can be used for more and/or other applications that could not be performed by prior art stimulators having a capacitive power source.

Processor 14 is coupled to the charge/discharge monitor 9. The charge/discharge monitor 9 sends signals to processor 14, and from these signals, processor 14 determines when a "fully charged" state is reached in the Li+ rechargeable power source 2. When processor 14 determines that a "fully charged" state is reached in the Li+ rechargeable power source 2, processor 14 then sends a signal via antenna 12 and onto the replenish transmitter 22. Upon receipt of this command signal, replenish transmitter 22 turns off the power transmitted to inductor 11.

The medical implantable stimulator of the present invention can be used as a neurostimulator or any other implantable system requiring self-contained power, e.g. pacemakers, defibrillators, and cochlear implants. The implantable stimulator of the present invention can provide up to at least 300 milliamp hours of stimulation. For smaller implantable stimulators, such as cochlear implants, the implantable stimulator of the present invention can provide up to at least 50 milliamp hours of stimulation.

In a preferred embodiment of the present invention, the implantable stimulator having a Li+ rechargeable power source can power a 12 cubic centimeter neurostimulator for up to about 30 days without recharging.

Another embodiment of the present invention provides an implantable stimulator having equivalent or better operating times than conventional capacitive powered stimulators while being smaller and lighter than such conventional capacitive powered stimulators.

Another preferred embodiment of the present invention provides an implantable stimulator that can be as small as three (3) to (6) cubic centimeters, and thus provide for anatomical placement of implantable stimulators not available in conventional capacitive powered stimulators.

The implantable stimulator of the present invention can be a neurostimulator such as Itrel® II, Itrel® 3, Synergy® and Kinetra® (all from Medtronic, Inc. of Minneapolis, Minnesota) that is modified so as to have a Li+ rechargeable power source, as opposed to a non-rechargeable power source currently used in such neurostimulators.

The charging and discharging of Li+ power source cell is a chemical reaction. Voltage is an indicator of the capacity of the power source cell related to the percentage electrical power charged or discharged.

Preferably, the Li+ power source cell of the implantable stimulator of the present invention is not overcharged or over discharged, while at the same time utilizing maximum energy. Over charging or over discharging the Li+ power source cell can adversely effect the parameters listed below, as well as permanently damage the Li+ power source cell:

> (1) total energy stored by each Li+ power source from full charge to full discharge;
> (2) capacity fade rate as a function of cycle;
> (3) capacity fade rate as a function of time;
> (4) capacity fade rate as a function of storage temperature;
> (5) charge rate capability; and
> (6) discharge current capability (impedance current).

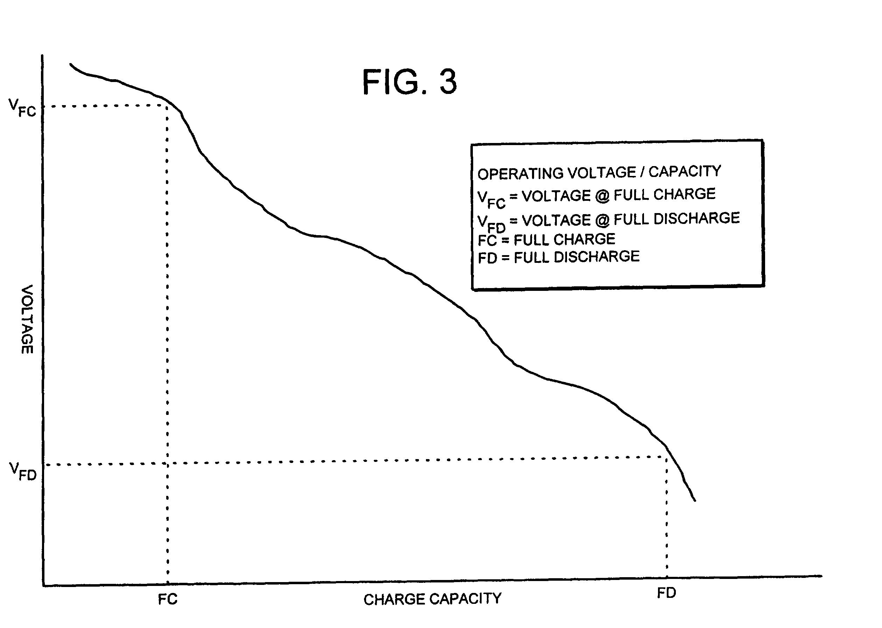

The Li+ power source cell charge/discharge shape, i.e., voltage versus charge capacity, for a typical Li+ power source cell is shown in Figure 3. It is preferable that the rate of charge and discharge be controlled and that the voltage of the Li+ power source cell be maintained between VFC (Voltage at full charge) and VFD (Voltage at full discharge) as identified in Figure 3 to prevent damage to the Li+ power source cell. To control the charge and discharge of the Li+ power source cell, the voltage of the Lit power source cell can be monitored by the charge/discharge monitor 9 in Figure 1, and controlled by processor 14, which receives signals from the charge/discharge monitor 9. It is further preferable to have the largest VFC to VFD possible.

Measuring the voltage of the Li+ power source cell as described above is one method to monitor the percentage of full charge and to monitor the rate of charge/discharge. However, there are effects that are not completely reflected in a voltage measurement. For example, the measurement of the voltage is not an instantaneous type of measurement, but takes some measurement response time. By the time a voltage measurement is made, it may be too late for processor 14 to properly control the rate of charge or discharge, and the Li+ power source cell may become damaged.

Another method of measuring in the present invention involves measuring the charge that is put into (charging) or removed from (discharge) the Li+ power source. This type of measurement can be referred to as a "coulomb counter". In this measurement method, processor 14 measures the voltage across a precision resistor (current) and integrates or samples over time to measure charge or coulombs. Processor 14 is coupled to the precision resistor (not shown) and to the Li+ power source. This coulomb counter method can be used alone or in combination with the voltage measurement of the power source cell described above. During replenishing, the coulomb counter can be considered to be a coefficient coupling feedback indicator.

## Claims

**1.** A medical system for delivering electrical stimulation pulses, in a controlled manner, to a targeted site within a patient comprising:

> an implantable stimulator (1) capable of delivering, in a controlled manner, one or more electrical stimulation pulses to a targeted site (4, 5, 6, 7) within a patient;
> the implantable stimulator having a rechargeable lithium ion power source (2) sufficient to supply power to the implantable stimulator on an exclusive basis.

**2.** The medical system of Claim 1 further comprising an inductor (11) adapted to gather EMF power transmissions and electrically coupled to recharge and regulation circuitry (10).

**3.** The medical system of Claim 2 further comprising a voltage regulator (8), a processor (14), telemetry and communication circuitry (13), and an antenna (12), where the voltage regulator is electrically coupled to the lithium ion power source (2) for detecting and issuing a low voltage command when a voltage at the lithium ion power source falls below a predetermined level;

the antenna (12) is electrically coupled to the telemetry and communication circuitry (13), and the telemetry and communication circuitry (13) is electrically coupled to the processor (14),
the processor (14) is electrically coupled to the telemetry and communication circuitry (13), the voltage regulator (8), the recharge and regulation circuitry (10), and at least one implanted lead (16);
the telemetry and communication circuitry (13) transmits a signal representative of the low voltage command to an electrical power replenisher, external to the implantable stimulator (1), to replenish the lithium ion power source (2) up to its maximum rated voltage by generating the EMF power transmissions near the inductor (11); the electrical power replenisher is capable of receiving the signal representative of the low voltage command and controlling the EMF power transmissions to the inductor of the implantable stimulator based on the command low voltage command.

**4.** The medical system of Claim 2 or 3 wherein the inductor (11) includes an inductor coil made of high permiable material.

**5.** The medical system of any preceding Claim wherein the implantable stimulator (1) has an inner housing (17) within an outer housing (18).

**6.** The medical system of Claim 5 wherein the inductor (11) is located outside the inner housing (17) and inside the outer housing (18).

**7.** The medical system of Claim 5 or 6 wherein the inner housing (17) is made from material comprising titanium or titanium alloys.

**8.** The medical system of Claim 3 wherein the implantable stimulator (1) has an inner housing (17) and an outer housing (18), and the antenna (12) coupled to the telemetry and communication circuitry (13) is located outside the inner housing and inside the outer housing.

**9.** The medical system of any of Claims 2 to 8 wherein the inductor (11) is sized large enough to facilitate the transfer rate due to the high energy stored in the lithium ion power source (2).

**10.** The medical system of any preceding Claim further including a processor to sample the voltage stored in the lithium ion power source (2), monitor the voltage over time, and control the addition or reduction of voltage stored in the lithium ion power source to control a charge capacity of the lithium ion power source.

**11.** The medical system of any preceding Claim further including a processor coupled to a precision resistor and to the lithium ion power source (2) to measure the electrical charge added or removed from the lithium ion power source to control a charge capacity of the lithium ion power source.

**12.** The medical system of any preceding Claim wherein the implantable stimulator (1) is between 3 to 6 cubic centimeters and provides about 4 days between replenishing of the lithium ion power source (2) having a depletion current of about 100 to 150 microamps.

**13.** The medical system of any of Claims 1 to 11 wherein the implantable stimulator (1) is between 6 to 12 cubic centimeters and provides about 30 days between replenishing of the lithium ion power source (2) having a depletion current of about 100 to 150 microamps.

**15.** The medical system of any of Claims 1 to 11 wherein the implantable stimulator (1) is between 12 to 26 cubic centimeters and provides about 60 days between replenishing of the lithium ion power source (2) having a depletion current of about 100 to 150 microamps.

FIG. 1

EP 1 048 324 A2

FIG. 2A    FIG. 2B    FIG. 2C    FIG. 2D

# FIG. 3

OPERATING VOLTAGE / CAPACITY
$V_{FC}$ = VOLTAGE @ FULL CHARGE
$V_{FD}$ = VOLTAGE @ FULL DISCHARGE
FC = FULL CHARGE
FD = FULL DISCHARGE